Europäisches Patentamt

**European Patent Office**

**Office européen des brevets**

(19)

(11) Veröffentlichungsnummer: **0 159 367**
A1

(12) # EUROPÄISCHE PATENTANMELDUNG

(21) Anmeldenummer: **84103754.2**

(22) Anmeldetag: **05.04.84**

(51) Int. Cl.⁴: **C 12 N 1/32**, C 12 N 1/20 // (C12N1/32, C12R1:26),(C12N1/20, C12R1:26)

(43) Veröffentlichungstag der Anmeldung: **30.10.85**
**Patentblatt 85/44**

(84) Benannte Vertragsstaaten: **BE DE FR GB IT NL**

(71) Anmelder: **BORSODI VEGYI KOMBINAT, Bolyai tér 1, H-3702 Kazincbarcika (HU)**

(72) Erfinder: **Holló, János, Prof. Dr., Guyon R. u. 9, Budapest II (HU)**
Erfinder: **Nyeste, László, Dr., Béla kir. u. 32, Budapest XII (HU)**
Erfinder: **Kirchknopf, László, Dr., Zólyom u. 6, Budapest XII (HU)**
Erfinder: **Zieger, Bertalan, Dipl. Chem., Szabó L.u.4. V/1, Kazincbarcika (HU)**
Erfinder: **Ballagi, András, Zsombolyai u.8, Budapest, XI (HU)**
Erfinder: **Kiss, Sándor A., Dr. Dipl. Chem., Lenin ut 28. I/4, Kazincbarcika (HU)**

(74) Vertreter: **Patentanwälte Beetz sen. - Beetz jun. Timpe - Siegfried - Schmitt-Fumian, Steinsdorfstrasse 10, D-8000 München 22 (DE)**

(54) **Neuer methanolassimilierender Methylomonas-Stamm und Verfahren zur Herstellung von Einzellerprotein auf der Basis von Methanol.**

(57) Die Erfindung betrifft den neuen methanolassimilierenden Bakterienstamm Methylomonas nov. spec. Nr. 7–3, der in der Stammsammlung beim Ungarischen Hygieneinstitut unter der Nr. 00196 sowie bei DSM, Göttingen, unter der Nr. DSM 2937 hinterlegt ist, sowie ein Verfahren zur Herstellung von mikrobiellem Protein durch Kultivierung dieses Stamms in Gegenwart von Methanol als einziger Kohlenstoff- und Energiequelle in einer anorganische Salze enthaltenden Nährlösung unter aeroben Bedingungen, ggf. Wärme- und/oder Alkalibehandlung der entstandenen Zellmasse und Isolierung der Zellmasse aus der Fermentationsbrühe. Der neue Methylomonas-Stamm liefert hohe Zellausbeuten bei kurzer Generationsdauer.

0159367

- 1 -

Neuer methanolassimilierender Methylomonas-
Stamm und Verfahren zur Herstellung von
Einzellerprotein auf der Basis von Methanol

Die Erfindung betrifft einen neuen methanolassimilierenden Methylomonas-Stamm und seine Verwendung zur Herstellung einer eiweißreichen Zellmasse durch Kultivierung in einem Methanol und anorganische Salze enthaltenden Nährmedium. Derartige
eiweißreiche Biomassen eignen sich in erster Linie
zur Tierfütterung, sind aber nach entsprechender
Reinigung auch zur menschlichen Ernährung geeignet.

Der gravierende, mit der Bevölkerungsexplosion
zusammenhängende, weltweite Eiweißmangel und dessen
Zusammenhänge zeigen die Schlüsselrolle von Eiweiß
in der Ernährung. Deshalb wurden in den letzten
Jahren außerordentlich breite und intensive Forschungsarbeiten zur Erschließung neuer Proteinquellen durchgeführt. Neue Perspektiven erschlossen
sich auch für die Herstellung von Einzellerprotein
durch Fermentation: Einerseits durch die mikrobiologische Umwandlung der in großen Mengen zur
Verfügung stehenden und sich erneuernde Cellulosebasis, andererseits durch Verwendung von unkonventionellen Substraten, von der landwirtschaftlichen

49-46871-1132-SF-Bk

Produktion unabhängigen Kohlenwasserstoffen und Kohlenwasserstoffderivaten.

Aufgrund experimenteller Erfahrungen ist heute bereits sicher, daß der Nutzung von Einzellerprotein auf der Basis von Kohlenwasserstoffen und Kohlenwasserstoffderivaten für Futterzwecke keinerlei toxikologische und ernährungsphysiologische Grenzen gesetzt sind; die praktische Anwendbarkeit hängt demnach von der Wirtschaftlichkeit der Technologie und dem Marktinteresse ab.

Die Wirtschaftlichkeit der Herstellung von Einzellerprotein durch Fermentation wird von zahlreichen Faktoren beeinflußt: Am wichtigsten sind die physiologischen und die die Kultivierung betreffenden Eigenschaften des verwendeten Mikroorganismus, sein Nährwert, die Art der eingesetzten Kohlenstoff- und Energiequelle, dh des Substrats, sowie die technologischen Anforderungen des dadurch bestimmten großtechnischen Fermentationsverfahrens, die in erster Linie die Sauerstoffversorgung, den Wärmeaustausch und Verarbeitungsfragen betreffen.

Gegenüber Substraten auf der Basis von Kohlenwasserstoffen und Kohlenwasserstoffderivaten ist vom Standpunkt der Fermentation die Anwendung von Methanol am vorteilhaftesten. Im Gegensatz zu Methan und den flüssigen Kohlenwasserstoffen sind bei Fermentationen auf der Basis von Methanol der Sauerstoffbedarf und die entstehende Wärme geringer; weiterhin ist Methanol unbeschränkt mit Wasser mischbar und kann leicht vom Produkt isoliert sowie in großer Reinheit hergestellt werden.

Von den Mikroorganismen sind Schimmelpilze, Hefen und Bakterien zur Nutzung von Methanol als einziger Kohlenstoffquelle geeignet. Bei Schimmeln und Hefen sind jedoch die Wachstumsgeschwindigkeit, die auf Methanol bezogene Zellenzahl und der Eiweißgehalt des Produkts bedeutend kleiner als bei den Bakterien.

Es sind bereits mehrere Bakterienarten bekannt, die für die Eiweißsynthese als Kohlenstoffquelle Methanol verwenden, ua Stämme von Pseudomonas sp. ATCC Nr. 21439 und ATCC Nr. 21438, Corynebacterium sp. ATCC Nr. 21232 und ATCC Nr. 21236 (DE-PS 2 059 277), Stämme von Pseudomonas rosea (AT-PS 324 997), der Stamm Methylomonas methanolica NRRL B-5458 (GB-PS 1 420 264), der Stamm Methylomonas espexii (GB-PS 1 480 684), die Stämme Pseudomonas utilis FERM-P 1690 und FERM-P 1691 sowie Pseudomonas inaudita FERM-P 1693, FERM-P 1694 (DE-PS 2 402 217).

Die Nachteile bei der Verwendung der obigen Bakterienstämme bestehen darin, daß ihre Generationsdauer lang und die erzielbare Ausbeute niedrig ist bzw die meisten der obigen Bakterienstämme nicht obligat methylotroph sind.

Die DE-PS 2 458 851 beschreibt für die Herstellung von Einzellerprotein den obligat Methanol nutzenden Stamm Methylomonas sp. DSM 580. Die Nachteile der Anwendung dieses Stamms liegen darin, daß die Ausbeute der Einzellerproteinproduktion niedrig ist (Y = 0,40 - 0,46) und ein schwach rosa gefärbtes Pigment entsteht, das bei der Nutzung als Nährstoff die Verdaulichkeit verschlechtert.

Für die Herstellung von Einzellerprotein auf der Basis einer Methanolkohlenstoffquelle ist der obligat Methanol nutzende Methylomonas probus FERM-P 3193 (DE-PS 2 708 112) bisher am besten geeignet. Die Generationsdauer dieses Stammes ist zwar kurz, jedoch ist die erzielbare Ausbeute niedrig (Y = 0,45 - 0,49).

Es wurde daher im Rahmen der Erfindung angestrebt, einen obligat Methanol nutzenden Stamm ausfindig zu machen, der auf Methanol bei außerordentlich guten Wachstumseigenschaften (kurze Generationsdauer) ein Produkt mit hohem Proteingehalt in hoher Ausbeute produziert, wobei weiterhin die entstehende Zellmasse leicht von der Fermentationsbrühe isoliert werden kann und die Aminosäurezusammensetzung des Produkts den Vorschriften der FAO entspricht, sowie ein entsprechendes Fermentationsverfahren anzugeben. Die Aufgabe wird anspruchsgemäß gelöst.

Aus natürlichen Quellen wurden mehrere methylotrophe Bakterienstämme isoliert, von denen einer der obigen Zielsetzung entsprach. Das isolierte Bakterium ist ein bisher unbekannter, noch nicht beschriebener, obligat methylotropher Bakterienstamm, der mit dem Namen Methylomonas nov. spec. Nr. 7-3 gekennzeichnet und beim Ungarischen Hygieneinstitut in der Ungarischen Nationalen Stammsammlung mit der Deponiernummer 00196 sowie der Deutschen Sammlung von Mikroorganismen (DSM), Göttingen, unter der Nr. DSM 2937 hinterlegt wurde.

Der Stamm Methylomonas nov. spec. Nr. 7-3 (DSM 2937) wird durch folgende Eigenschaften gekennzeichnet:

## Morphologische Eigenschaften

In einer flüssigen MSM-Nährlösung (Methods in Microbiol. Vol. 3/b) mit einem Methanolgehalt von 1 Vol-% können bei einer Temperatur von 30 °C nach 24 h Züchtung, stäbchenförmige Zellen einer mittleren Größe von (0,5-1) x (1-2) μm beobachtet werden. Die Zellen bewegen sich mit Hilfe eines polaren Flagellums, im allgemeinen allein, aber oft auch zu zweit und bilden manchmal zu dritt verflochten eine Kette. Der Stamm ist Gram-negativ. Sich auf festem MSM-Nährboden entwickelnde Kolonien besaßen im Alter von zwei Tagen eine glatte Oberfläche; ihr Durchmesser betrug 0,5 - 1 mm; sie waren farblos, mit gezacktem Rand. 3 bis 5 Tage alte Kolonien besitzen einen großen, farblosen glatten Hof. Ihr Durchmesser beträgt etwa 2 - 2,5 mm; ihr Rand ist 'fjordartig' ausgebildet; die Mitte der Kolonie ist schwach beige-braun, ihre Oberfläche bläulich.

## Physiologische und das Wachstum betreffende Eigenschaften

Der isolierte Stamm Methylomonas nov. spec. Nr. 7-3 wächst nur auf methylotrophen Substranten: Auf Methanol sehr gut, auf Dimethylamin und Formaldehyd nur schwach; Methan wird nicht genutzt. Auf anderen eine C-C-Bindung enthaltenden Substraten, wie verschiedenen Zuckern, organischen Säuren, Aminosäuren, Alkoholen, Kohlenwasserstoffen, wurde kein Wachstum beobachtet.

Von den Stickstoffquellen wächst er am besten auf Ammoniumsalzen; Nitrate, Harnstoff, Casein-(Caseinhydrolysat)
aminosäuren/und Hefeextrakt werden schwächer genutzt.

Das Wachstum des Stamms wird durch Wachstumsfaktoren (Biotin, Thiamin, Hefeextrakt) nicht
stimuliert.

Die Temperatur des optimalen Wachstums liegt zwischen 33 und 36 °C, jedoch kann auch bei 25 bzw 39 °C
noch Wachstum beobachtet werden. Der für das Wachstum optimale pH-Wert liegt zwischen 6,5 und 7,5;
der Stamm vermehrt sich aber auch noch bei pH-Werten von 5,5 bzw 9.

Die maximale Wachstumsgeschwindigkeit kann bei
einer Methanolkonzentration unter 0,6 Volumprozent
erzielt werden.

Die höchste spezifische Wachstumsrate des Stamms
beträgt aufgrund von kontinuierlichen Kultivierungsversuchen im Chemostaten $\mu_{max} = 0,691$ h$^{-1}$ (Generationsdauer etwa 1 h). Die beste erreichte Ausbeute war
hingegen Y = 0,55 g Zellen/g Methanol.

In einer submersen Kultur ist die Grundfarbe der
Kultur gelblich-weiß, im Sauerstofflimit grünlich,
in einer Nährlösung ohne Kohlenstoffquelle nach einigen Stunden ohne Nahrungsangebot hat sie einen
bräunlichen Farbton.

Die Wirkung verschiedener Antibiotica auf das
Wachstum des Stamms ist in Tabelle 1 veranschaulicht.

Tabelle 1

| Antibioticum | Menge | Wirkung auf das Wachstum | Durchmesser der Hemmzone |
|---|---|---|---|
| Penicillin | 3 NE | Ø | |
| Oxacillin | 10 μg | Ø | |
| Methicillin | 20 μg | Ø | |
| Chloramphenicol | 30 μg | Ø | |
| Oleandomycin | 30 μg | Ø | |
| Streptomycin | 30 μg | Ø | |
| Tetracyclin | 30 μg | - | 2,5 cm |
| Oxytetracyclin | 30 μg | - | 3 cm |
| Chlortetracyclin | 30 μg | - | 5 cm |
| Neomycin | 100 μg | Ø | |
| Polymycin B | 15 μg | - | 1 cm |
| Erythromycin | 10 μg | - | 1 cm |
| Vancomycin | 50 μg | Ø | |
| Kanamycin | 30 μg | - | 1,3 cm |
| Spiramycin | 30 μg | Ø | |
| Novobiocin | | | |
| Ampicillin | 20 μg | Ø | |
| Colistin | 20 μg | Ø | |
| Lincomycin | 10 μg | Ø | |
| Nevigramon | 30 μg | - | 1,5 cm |
| Gentamycin | 20 μg | - | 1 cm |
| Carbenicillin | 50 μg | Ø | |

Abkürzungen:

Ø :    Wachstum wird nicht beeinflusst

- :    Wachstum wird gehemmt

Die biochemischen Eigenschaften des Stamms
Methylomonas nov. spec. Nr. 7-3 (DSM 2937)
werden durch die folgenden Ergebnisse biochemischer
Tests charakterisiert:

| | |
|---|---|
| Hexulosephosphat-Synthetase-(HPS-)-Aktivität | + |
| Hydroxypyruvat-Reductase-(HPR-)-Aktivität | - |
| Nitratassimilation | + |
| Reduktion des Nitrats zu Nitrit | + |
| Indolproduktion auf MeOH | - |
| Stärkehydrolyse | - |
| $H_2S$-Produktion | - |
| Gelatinezerfließen | - |
| Acetonproduktion | + |
| Wachstumsfaktorbedarf | - |
| Ureaseproduktion | + |
| Katalaseproduktion | + |
| Halotoleranz | 3 %. |

Der den Gegenstand der Erfindung bildende, oben
beschriebene Bakterienstamm gehört aufgrund seiner
morphologischen, wachstumsmäßigen, physiologischen
bzw biochemischen Eigenschaften nach BERGEY's
MANUAL of DETERMINATIVE BACTERIOLOGY, 8th Edition
(edited by Buchana, Gibbons, Cowan, Holt, Liston
Murray, Niven, Ravim and Starmer; The Williams and
Wilkins Company) zur Gattung Methylomonas. Nach
dem Handbuch von BERGEY gehören zum Genus Methylomonas drei Populationen: Methylomonas mechanica,
Methylomonas methanooxidans und Methylomonas
methanitrificans. Das erfindungsgemäße Bakterium
unterscheidet sich von den obigen drei Methylomonas-Populationen und auch von den in der Fachliteratur bisher beschriebenen, Methanol nutzenden Bakterien in den morphologischen Eigenschaften, der Nutzung der Kohlenstoffquelle, der Pigmentbildung, der Bildung von extracellulärem Schleim. sowie in der optimalen Temperatur des Wachstums und den kinetischen Wachstumseigenschaften. Das erfindungsgemäße Bakterium ist hinsichtlich der Generationsdauer fast identisch mit den bisher beschriebenen Methanol assimilierenden Bakterienstämmen,führt jedoch zu einer demgegenüber höheren auf Methanol bezogenen Ausbeute.

Die Verarbeitung der Fermentationsbrühe und die Gewinnung der Zellmasse kann durch Filtration, Sedimentation, Zentrifugieren, Flockungs- und/oder Flotationsverfahren und zweckmäßig Kombinationen solcher Verfahren erfolgen. Besonders vorteilhaft ist es, die Fermentationsbrühe vor der Gewinnung der Zellmasse einer Alkali- und Wärmebehandlung zu unterwerfen, wodurch der Nucleinsäuregehalt des Produkts von 14 - 15 % auf etwa 9 % sinkt. Hierdurch wird die Verdaulichkeit verbessert.

Das erfindungsgemäße Verfahren wird im folgenden anhand von Ausführungsbeispielen veranschaulicht.

Die Zusammensetzung der Fermentationsnährlösung entsprach in jedem Fall folgenden Salzkonzentrationen:

| | (g) |
|---|---|
| $(NH_4)_2SO_4$ | 5 |
| $CaCl_2 \cdot H_2O$ | 0,1 |
| $MgSO_4 \cdot 7H_2O$ | 0,1 |
| $KH_2PO_4$ | 4,7 |
| $Na_2HPO_4 \cdot 12H_2O$ | 13,0 |

und Spurenelemente.

Die Menge des Bakteriums betrug in jedem Fall 2 Vol.-% (in einem Inoculum mit einem Zellengehalt von 4 g/l).

Beispiel 1

Diskontinuierliche Fermentation in einem Fermenter:

Bedingungen:

| | |
|---|---|
| Nutzinhalt: | 6 l |
| Belüftungsrate: | 0,5 Vol./Vol.·min<br>(Volumen Luft/<br>Volumen Fermenta-<br>tionsbrühe pro min) |
| Rührerdrehzahl: | 700 $min^{-1}$. |

In dem gegebenen Fermenter betrug die Sauerstoffübertragungsrate unter den von den obigen Parametern bestimmten Bedingungen aufgrund der Messung nach der Sulfitoxidationsmethode 90 mmol $O_2$/l·h.

Anfängliche Methanolkonzentration: 1 Vol.-%.

Temperatur: 30 °C pH-Wert: Durch Zusetzen von 10-%igem $NH_4OH$ wurde der pH-Wert zwischen 6,7 und 7,2 gehalten.

Ergebnisse:

Das Methanol (1 Vol.-%) wurde in 9 h verbraucht. Die Fermentationsbrühe wurde bei einem pH-Wert zwischen 7 und 8 15 min lang bei einer Temperatur zwischen 45 und 65 °C einer Wärmebehandlung unterworfen; dann wurde der pH-Wert auf 3,5 eingestellt. Die ausgeschiedene Zellmasse wurde abdekantiert und versprüht auf einem Trockner getrocknet. Hierbei wurden 3,69 g Zelltrockenmasse pro Liter Fermentationsbrühe erhalten. Die auf die gesamte Fermentation berechnete Ausbeute betrug 0,4659 g Zellen/g MeOH. Die bei der Fermentation meßbare kürzeste Generationsdauer betrug 1,4 h.

Beispiel 2

Diskontinuierliche Fermentation in einem Tauchstrahlfermenter (Jet-Typ)

Bedingungen:

Nutzinhalt:                  130 1
Belüftungsverhältnisse: Sauerstoffübertragungsrate unter den gegebenen Parametern 450 mmol $O_2/l \cdot h$ (Sulfidoxidationsmethode).
pH-Wert:                  Mit $NH_4OH$ zwischen 6,6 und 6,9 gehalten.
Temperatur:              Zwischen 29 und 35 °C.
Anfängliche Methanolkonzentration: 6 g/l.

Die Fermentationsbrühe wurde wie in Beispiel 1 verarbeitet.

Ergebnisse:

Das auf einmal zugesetzte Methanol wurde in 3,5 h verbraucht. Es entstand eine Zellmasse von 381,4 g. Die für die gesamte Fermentation berechnete Ausbeute betrug Y = 0,48 g Zellen/g MeOH.

Beispiel 3

Kontinuierliche Fermentationsversuche

Die kontinuierlichen Fermentationsversuche erfolgten in einem Fermenter mit einem Nutzinhalt von 6 1. Die Fermentationsbrühe wurde wie in Beispiel 1 verarbeitet.

Belüftungsverhältnisse: 1 Vol./Vol.·min (Belüftungsrate)
Rührerdrehzahl: 900 $min^{-1}$.

Die Zusammensetzung der Nährlösung entsprach der oben angegebenen; die Methanolkonzentration betrug 2 g/l.

Ergebnisse von bei unterschiedlichen pH- und Temperaturwerten durchgeführten kontinuierlichen Kultivierungsversuchen:

3.1.1 pH = 6,5; T = 30 °C, $\mu_{max}$ = 0,683 $h^{-1}$;

D = 0,587 $h^{-1}$: Bei dieser Verdünnungsrate betrug die Zellenkonzentration im stationären Zustand $\bar{x}$ = 1,2 g/l.

Ausbeute: Y = 0,55 g Zellen/g MeOH;

3.1.2 D = 0,66 $h^{-1}$: Bei dieser Verdünnungsrate betrug die Zellenkonzentration im stationären Zustand $\bar{x}$ = 1,07 g/l.

Ausbeute: Y = 0,52 g Zellen/g MeOH.

3.2.1 pH = 7; T = 30 °C, $\mu_{max}$ = 0,565 $h^{-1}$;

D = 0,51 $h^{-1}$: Bei dieser Verdünnungsrate betrug die Zellenkonzentration im stationären Zustand

$\bar{x}$ = 1,06 g/l.

Ausbeute: Y = 0,48 g Zellen/g MeOH.

3.2.2 D = 0,56 $h^{-1}$: Bei dieser Verdünnungsrate betrug die Zellenkonzentration im stationären Zustand

$\bar{x}$ = 0,25 g/l.

Ausbeute: Y = 0,53 g Zellen/g MeOH.

3.3.1 pH = 7; T = 33 °C; $\mu_{max}$ = 0,691 $h^{-1}$;

D = 0,575 $h^{-1}$: Bei dieser Verdünnungsrate betrug die Zellenkonzentration im stationären Zustand

$\bar{x}$ = 0,96 g/l.

Ausbeute: Y = 0,44 g Zellen/g MeOH.

3.3.2 D = 0,66 $h^{-1}$: Bei dieser Verdünnungsrate betrug die Zellenkonzentration im stationären Zustand

$\bar{x}$ = 0,56 g/l.

Ausbeute: Y = 0,43 g Zellen/g MeOH.

Beispiel 4:

Vergleich der Zellenzusammensetzung des mit dem Verfahren nach Beispiel 2 hergestellten Produkts mit der Zellenzusammensetzung handelsüblicher Produkte (Pruteen, ICI, und Probion, Hoechst).

Die Ergebnisse sind in Tabelle 2 zusammengefaßt.

Tabelle 2

| | % Aminosäuren im Produkt | | |
|---|---|---|---|
| | Pruteen | Probion | Produkt v. Beispiel 2 |
| Lysin | 4,6 | 4,9 | 5,0 |
| Methionin | 1,8 | 2,1 | 1,8 |
| Cystin | 0,5 | | 0,9 |
| Threonin | 3,5 | 3,5 | 3,6 |
| Tryptophan | 1,0 | 1,6 | nicht bestimmt |
| Leucin | 5,3 | 5,6 | 5,8 |
| Valin | 3,5 | 3,5 | 3,3 |
| Tyrosin | 4,2 | | 5,0 |
| Phenylalanin | 2,7 | 3,2 | 3,4 |
| Histidin | 1,3 | 1,4 | 1,8 |
| Arginin | 3,5 | 2,5 | 4,4 |
| Alanin | 5,1 | 5,4 | 5,9 |
| Asparaginsäure | 6,5 | 7,0 | 9,4 |
| Glutaminsäure | 7,7 | 6,8 | 9,3 |
| Glycin | 4,0 | 4,2 | 4,5 |
| Prolin | 2,4 | 3,1 | 2,9 |
| Serin | 2,3 | 2,7 | 4,2 |
| | 62,2 | 60,0 | 69,6 |
| Nucleinsäuren | 12 | nicht bestimmt | 9 |

Ansprüche

1. Bakterienstamm Methylomonas nov. spec. Nr. 7-3, hinterlegt in der Ungarischen Stammsammlung beim Ungarischen Hygieneinstitut unter der Nr. 00196 sowie bei DSM, Göttingen, unter der Nr. DSM 2937.

2. Verfahren zur mikrobiellen Proteinherstellung durch Kultivierung eines Bakterienstamms der Gattung Methylomoras in Gegenwart von Methanol in einer anorganische Salze enthaltenden Nährlösung unter aeroben Bedingungen und Isolierung der Zellmasse aus der Fermentationsbrühe, gekennzeichnet durch Verwendung des Stamms Methylomonas nov. spec. Nr. 7-3, der in der Ungarischen Stammsammlung beim Ungarischen Hygieneinstitut unter der Nr. 00196 sowie bei DSM, Göttingen, unter der Nr. DSM 2937 hinterlegt ist.

3. Verfahren nach Anspruch 2, dadurch gekennzeichnet, daß die Kultivierung bei einer Temperatur zwischen 30 und 37 °C vorgenommen und der pH-Wert der Fermentationsbrühe zwischen 6 und 8 gehalten wird.

49-46871-1132-SF-Bk-Ms

4. Verfahren nach Anspruch 2 oder 3, dadurch gekennzeichnet, daß die Verarbeitung der Fermentationsbrühe und die Gewinnung der Zellmasse durch Filtration, Zentrifugieren, Flockung und/oder Flotation vorgenommen wird.

5. Verfahren nach einem der Ansprüche 1 bis 4, dadurch gekennzeichnet, daß die erhaltene Zellmasse vor der Abtrennung einer Wärme- und/oder Alkalibehandlung unterworfen wird.

EUROPÄISCHER RECHERCHENBERICHT

0159367

Nummer der Anmeldung

EP 84 10 3754

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 100, Nr. 11, 12. März 1984, Seite 429, Nr. 84214s, Columbus, Ohio, US; NYESTE, LASZLO: "Optimization of fermentation processes" & SZESZIPAR 1983, 30(4), 130-3 * Insgesamt * | 1-5 | C 12 N  1/32<br>C 12 N  1/20 //<br>(C 12 N  1/32<br>C 12 R  1/26 )<br>(C 12 N  1/20<br>C 12 R  1/26 ) |
| | --- | | |
| X | CHEMICAL ABSTRACTS, Band 100, Nr. 25, 18. Juni 1984, Seite 441, Nr. 207861k, Columbus, Ohio, US; NYESTE, LASZLO: "Optimization of fermentation processes" & SZESZIPAR 1982, 30(4), 130-3 * Insgesamt * | 1-5 | |
| | ----- | | |

RECHERCHIERTE SACHGEBIETE (Int. Cl.4)

C 12 N
C 12 R

Der vorliegende Recherchenbericht wurde für alle Patentansprüche erstellt.

| Recherchenort<br>DEN HAAG | Abschlußdatum der Recherche<br>06-12-1984 | Prüfer<br>DESCAMPS J.A. |
|---|---|---|